# DEMANDE DE BREVET EUROPEEN

(11) **EP 4 085 878 A1**
(43) Date de publication de la demande: **09.11.2022**
(21) Numéro de dépôt: 22172190.5
(22) Date de dépôt: 06.05.2022
(51) Int. Cl.: A61F 5/01, A61F 13/04, A61G 7/075

(54) **DISPOSITIF DE PROTECTION D'UN PIED ET PROCÉDÉ DE FABRICATION DE CE DISPOSITIF**

(30) Priorité: 07.05.2021 FR 2104883
(71) Demandeur: Roig, Agnès, 34230 Pouzols (FR)
(72) Inventeur: Roig, Agnès, 34230 Pouzols (FR)
(74) Mandataire: Cornuejols, Georges

(57) **Abrégé**

Le dispositif (20) de protection d'un pied (32) de dimensions prédéterminées, notamment contre des escarres, comporte une coque en matériau élastiquement déformable à mémoire de forme, coque dotée d'un volume intérieur vide (31). Débouchant dans ce volume, se trouvent :
- une ouverture (21) dite « de jambe » propre à envelopper la base de la jambe au-dessus des chevilles et
- une ouverture (23) dite « d'orteils » propre à laisser passer les cinq orteils du pied,
- une ouverture (22) dite « de talon » dont au moins une section présente une forme globalement triangulaire dont un coin est orienté vers l'ouverture de jambe et
- deux ouvertures (24) dites « de malléoles » de part et d'autre d'un plan passant par les ouvertures de jambe, d'orteils et de talon.

Les ouvertures de jambe et d'orteils sont reliées par des lèvres (33) disjointes, la coque étant déformable sans déformation définitive jusqu'à un écartement des lèvres d'au moins six centimètres sur toute leur longueur.

## Description

### DOMAINE TECHNIQUE DE L'INVENTION

La présente invention vise un dispositif de protection d'un pied et un procédé de fabrication de ce dispositif. Elle s'applique, en particulier, au domaine des appareils et accessoires de soin ou de protection corporelle ou médicale. Elle intéresse plus particulièrement à la protection du talon à des fins curatives ou préventives de l'escarre du talon et/ou à la protection des malléoles évitant en outre le syndrome équin et ce dans diverses positions du décubitus.

### ÉTAT DE LA TECHNIQUE

On connaît divers dispositifs destinés au repos et à la protection du pied et de la partie inférieure de la jambe, applicables notamment aux personnes immobilisées en position horizontale pour une période de longue durée. D'une façon générale, ces dispositifs visent à éviter des contacts prolongés des parties du pied en saillie. Ces contacts prolongés peuvent, en effet, provoquer la formation d'escarres, la présence et la persistance de ces escarres entraînant des coûts de soins élevés et constituant un problème majeur dans le cadre des budgets affectés à la santé publique.

On connaît ainsi des talonnières dites enveloppantes telles en peau de mouton, à microfibres à garniture gélifiée ou microbilles, permettant de réduire le point de pression ; mais ces accessoires sont peu confortables car ils nécessitent la mise en place de sangles de maintien. De plus, ils n'assurent pas de position stable du pied et ne protègent pas de l'équinisme.

On connaît aussi des talonnières du type gouttière ne permettant que le décubitus dorsal et nécessitant l'immobilité stricte des membres inférieurs. De plus, ces talonnières n'étant pas solidaires du pied, elles peuvent se révéler instables sous le pied. D'autres talonnières, plates, ne s'adaptent pas à toutes les positions et n'évitent pas l'équinisme.

On connaît le document US 5 827 211, qui décrit une botte orthopédique pouvant être ajusté autour d'un pied pour permettre un soutien et une protection de la peau et des tissus sous-jacents. Le dispositif entoure le pied et présente des ouvertures effilées dans lesquelles s'étendent les protubérances osseuses. L'épaisseur du corps du dispositif dépasse l'étendue des protubérances osseuses, réduisant ainsi le risque d'abrasion ou d'ulcération de la peau recouvrant les protubérances osseuses.

On connaît le document WO2009147316, qui décrit un dispositif de soin et de protection du pied, destiné à prévenir et gérer la formation de plaies chez les personnes contraintes de rester allongées pendant une période prolongée. Ce coussin d'appui est composé d'un bloc en matériau élastiquement déformable qui a la forme générale d'un anneau ou d'un tore avec une rainure et qui est traversé par une pluralité de canaux d'axe radial situé dans le plan équatorial du volume toroïdal. Le coussin comporte une fente disposée dans le plan équatorial et s'étendant le long d'un segment du volume toroïdal.

Les formes des dispositifs décrits dans ces documents peuvent provoquer un inconfort de l'utilisateur et une érosion cutanée, voire des plaies, notamment autour du tendon d'Achille.

D'une façon générale ces talonnières de type connu si elles ne sont pas exactement positionnées et maintenues, ce qui pose des problèmes pour des patients agités, peuvent engendrer des effets secondaires, tels que l'apparition d'escarres en dehors de la zone protégée, des blessures, contusions ou cisaillements.

### EXPOSE DE L'INVENTION

La présente invention vise à remédier à tout ou partie de ces inconvénients.

À cet effet, selon un premier aspect, la présente invention vise un procédé de fabrication d'un dispositif de protection d'un pied de dimensions prédéterminées, notamment contre des escarres, qui comporte une étape de moulage d'une coque, entre deux demi-moules externes correspondant aux surfaces extérieures du dispositif, et autour d'un moule interne correspondant à un volume intérieur vide du dispositif et comportant jusqu'au contact avec le moule externe :
- une branche dite « de jambe » propre à envelopper la base de la jambe au-dessus des chevilles,
- une branche dite « d'orteils » propre à laisser passer les cinq orteils du pied, les branches de jambe et d'orteils étant reliées par une nervure de séparation de lèvres, nervure qui rejoint les moules externes,
- une branche dite « de talon » dont au moins une section présente une forme globalement triangulaire dont un coin est orienté vers l'ouverture de jambe et
- deux branches dites « de malléoles » de part et d'autre d'un plan passant par les branches de jambe, d'orteils et de talon ;
le moulage de la coque se faisant dans un matériau élastiquement déformable à mémoire de forme.

La forme triangulaire de l'ouverture de talon permet une meilleure répartition des forces autour du talon d'Achille, celui-ci venant dans le coin de la forme triangulaire orienté vers l'ouverture de la jambe. Le procédé objet de l'invention permet de réaliser, en une seule étape de moulage, un dispositif de protection d'un pied. Ce dispositif dégage et protège les zones à risques telles la face arrière du talon, les malléoles, la base du gros orteil, des pressions génératrices d'escarres et ce dans les diverses positions de l'utilisateur. De plus, le dispositif protège le pied immobilisé contre le risque d'équinisme.

Dans des modes de réalisation, le matériau élastiquement déformable est configuré pour que la coque soit déformable sans déformation définitive jusqu'à un écartement des lèvres suffisant pour le passage du moule interne entre ces lèvres

On note que l'homme du métier sait configurer le matériau élastiquement déformable, sa densité dans le cas d'une mousse à cellules ouvertes, et son épaisseur en fonction de la déformation maximale ainsi définie, sur la base de ses connaissances générales des matières plastiques élastiques et des mousses, notamment de mousses à cellules ouvertes de matières synthétiques telles que le polyuréthane. Par exemple une mousse de polyuréthane (« PU ») de densité comprise entre 35 et 40 kg/m³ permet d'atteindre les spécifications du procédé objet de l'invention.

Dans des modes de réalisation, lors de l'étape de moulage, la coque formée est symétrique par rapport à un plan passant par les ouvertures de jambe, d'orteils et de talon.

Ainsi, le dispositif peut être adapté, de manière indifférenciée, au pied droit comme au pied gauche.

Dans des modes de réalisation, le procédé comporte, de plus, une étape de confection d'une housse et une étape d'assemblage de la coque et la housse, la housse étant enveloppante, amovible et configurée pour entourer la coque en laissant accessible au moins l'ouverture de jambe et l'ouverture d'orteils.

Dans des modes de réalisation, lors de l'étape de moulage, l'épaisseur des lèvres formées, mesurée dans un plan passant par les ouvertures de jambe, d'orteils et de talon, est inférieure à trois centimètres.

Dans des modes de réalisation, lors de l'étape de moulage, la coque formée présente deux méplats atteignant l'ouverture de talon, l'intersection des méplats définissant une première arête et l'intersection de chaque méplat et de l'ouverture de talon définissant deux autres arêtes plus courtes que la première arête, les trois arêtes formant un trièdre dont le sommet est entre l'ouverture de talon et l'ouverture de jambe et sensiblement à équidistance du centre de l'ouverture de jambe et du centre de l'ouverture d'orteils.

Dans des modes de réalisation, lors de l'étape de moulage, les méplats formés présentent, en dehors des arêtes, une périphérie sensiblement elliptique, dont l'axe du plus grand diamètre est dans un plan passant par l'ouverture de jambe et par l'ouverture de talon.

Dans des modes de réalisation, lors de l'étape de moulage, les ouvertures de malléoles formées sont cylindriques sur au moins une partie de la largeur de la coque.

Dans des modes de réalisation, le matériau élastiquement déformable à mémoire de forme est une mousse à cellules ouvertes de masse volumique comprise entre 30 et 40 kg/m3.

Dans des modes de réalisation, le procédé comporte, de plus, une étape de projection d'une peinture élastique sur la coque formée, la peinture élastique étant configurée pour adhérer à la mousse de la coque et fermer les cellules de surface de la mousse de la coque.

Dans des modes de réalisation, le procédé comporte une étape de dépôt d'une peinture thermochromique qui comporte un adjuvant dont la couleur varie avec la température.

Dans des modes de réalisation, au cours de l'étape de moulage, la mousse est thermochromique qui comporte un adjuvant dont la couleur varie avec la température.

Selon un deuxième aspect, la présente invention vise un dispositif moulé de protection d'un pied, qui comporte une coque en matériau élastiquement déformable à mémoire de forme, coque dotée d'un volume intérieur vide et, débouchant dans ce volume :
- une ouverture dite « de jambe » propre à envelopper la base de la jambe au-dessus des chevilles,
- une ouverture dite « d'orteils » propre à laisser passer les cinq orteils du pied, les ouvertures de jambe et d'orteils étant reliées par des lèvres disjointes, la coque étant déformable élastiquement sans déformation définitive jusqu'à un écartement des lèvres d'au moins six centimètres sur toute leur longueur,

- une ouverture dite « de talon » dont au moins une section présente une forme globalement triangulaire dont un coin est orienté vers l'ouverture de jambe et
- deux ouvertures dites « de malléoles » de part et d'autre d'un plan passant par les ouvertures de jambe, d'orteils et de talon.

Dans des modes de réalisation, la coque est symétrique par rapport à un plan passant par les ouvertures de jambe, d'orteils et de talon.

Dans des modes de réalisation, le dispositif comporte, de plus, une housse enveloppante amovible configurée pour entourer la coque en laissant accessible au moins l'ouverture de jambe et l'ouverture d'orteils.

Dans des modes de réalisation, l'épaisseur des lèvres, mesurée dans un plan passant par les ouvertures de jambe, d'orteils et de talon, est inférieure à trois centimètres.

Dans des modes de réalisation, la coque présente deux méplats atteignant l'ouverture de talon, l'intersection des méplats définissant une première arête et l'intersection de chaque méplat et de l'ouverture de talon définissant deux autres arêtes plus courtes que la première arête, les trois arêtes formant un trièdre dont le sommet est entre l'ouverture de talon et l'ouverture de jambe et sensiblement à équidistance du centre de l'ouverture de jambe et du centre de l'ouverture d'orteils.

Dans des modes de réalisation, les méplats présentent, en dehors des arêtes, une périphérie sensiblement elliptique, dont l'axe du plus grand diamètre est dans un plan passant par l'ouverture de jambe et par l'ouverture de talon.

Dans des modes de réalisation, les ouvertures de malléoles sont cylindriques sur au moins une partie de la largeur de la coque.

Dans des modes de réalisation, la coque est constituée d'une mousse à cellules ouvertes de masse volumique comprise entre 30 et 40 kg/m3.

Dans des modes de réalisation, la coque est traitée par une peinture élastique configurée pour adhérer à la mousse de la coque et fermer les cellules de surface de la mousse de la coque.

Dans des modes de réalisation, la peinture est thermochromique et comporte un adjuvant dont la couleur varie avec la température.

Dans des modes de réalisation, la mousse formant la coque, est thermochromique et comporte un adjuvant dont la couleur varie avec la température.

Les avantages, buts et caractéristiques particulières de ce dispositif étant similaires à ceux du procédé objet de l'invention, ils ne sont pas rappelés ici.

### BREVE DESCRIPTION DES FIGURES

D'autres avantages, buts et caractéristiques particulières de l'invention ressortiront de la description non limitative qui suit d'au moins un mode de réalisation particulier du dispositif et du procédé objets de la présente invention, en regard des dessins annexés, dans lesquels :
La figure 1 représente, schématiquement, en coupe verticale de côté, une coque d'un premier mode de réalisation particulier du dispositif objet de l'invention,
La figure 2 représente, schématiquement, en coupe verticale, le positionnement d'un pied dans la coque illustrée en figure 1,
La figure 3 représente, schématiquement, une coupe A-A de la coque, coupe repérée en figure 1,
La figure 4 représente, schématiquement, une coupe B-B de la coque, repérée en figure 1,
La figure 5 représente, schématiquement, une vue de dessous de la coque, selon une direction C repérée en figure 1,
La figure 6 représente, schématiquement, une vue de dessus de la coque illustrée en figures 1 à 5 entourée d'une housse, selon une direction D repérée en figure 1, la coque et la housse constituant un deuxième mode de réalisation particulier du dispositif objet de l'invention,
La figure 7 représente, schématiquement, une vue de face du dispositif illustré en figure 6, selon une direction E repérée en figure 1,
La figure 8 représente, schématiquement, une vue de dessus du dispositif illustré en figures 6 et 7, selon une direction F repérée en figure 1,
La figure 9 représente, sous forme d'un logigramme, des étapes d'un premier mode de réalisation particulier du procédé objet de l'invention, et
La figure 10 représente, sous forme d'un logigramme, des étapes d'un deuxième mode de réalisation particulier du procédé objet de l'invention.

### DESCRIPTION DES MODES DE REALISATION

La présente description est donnée à titre non limitatif, chaque caractéristique d'un mode de réalisation pouvant être combinée à toute autre caractéristique de tout autre mode de réalisation de manière avantageuse.

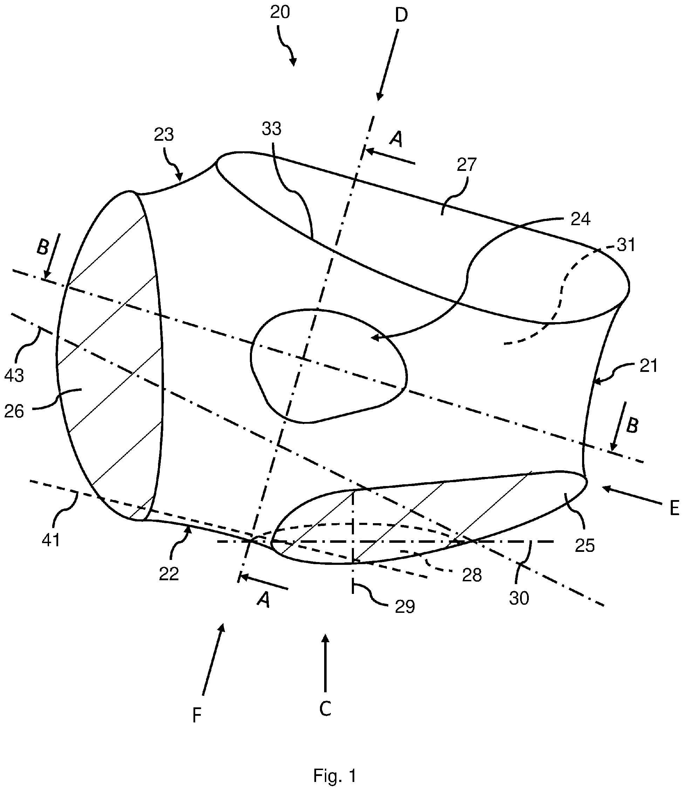

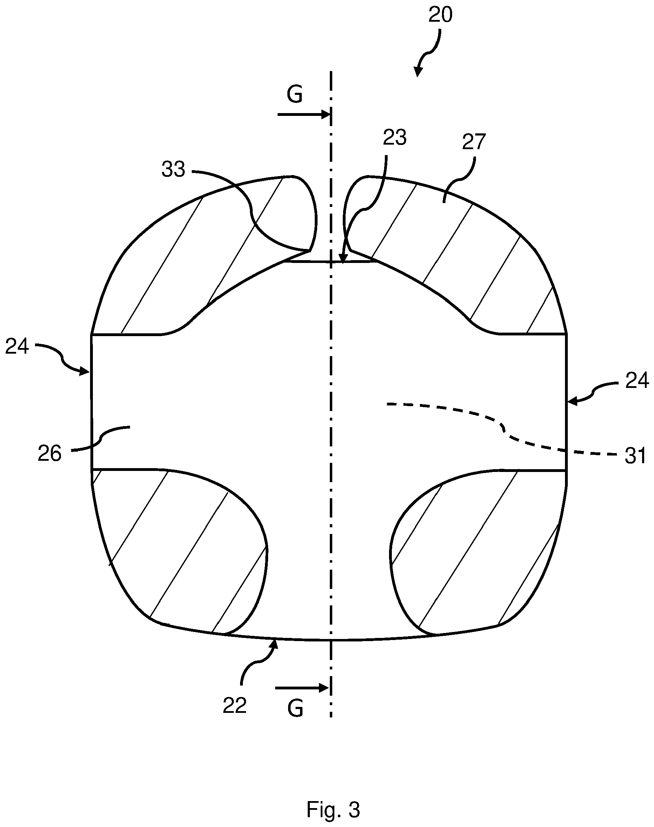

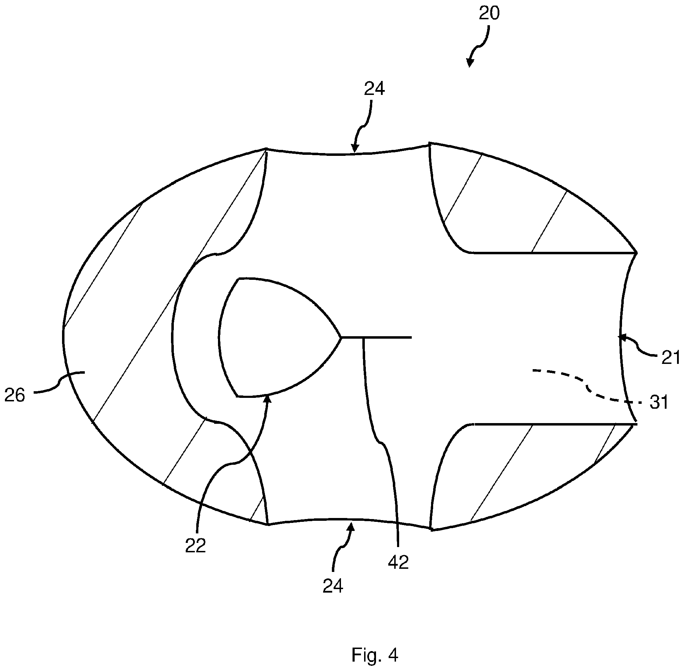

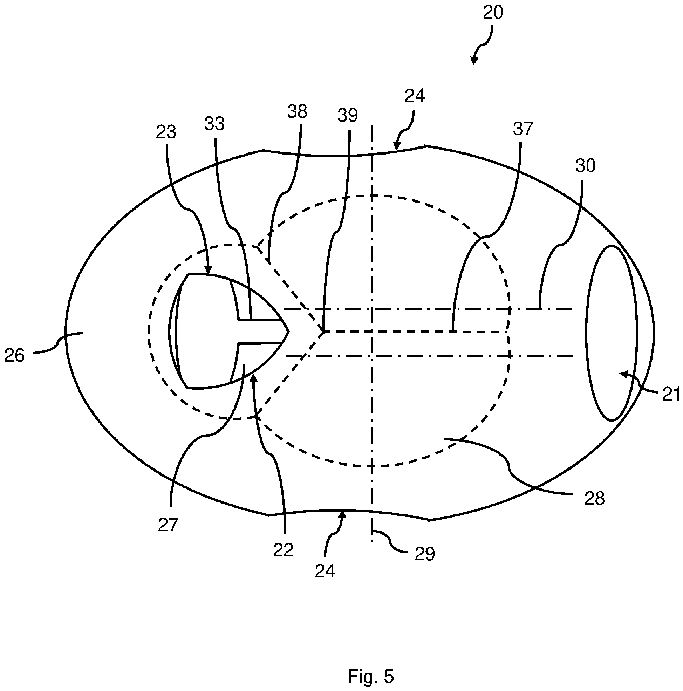

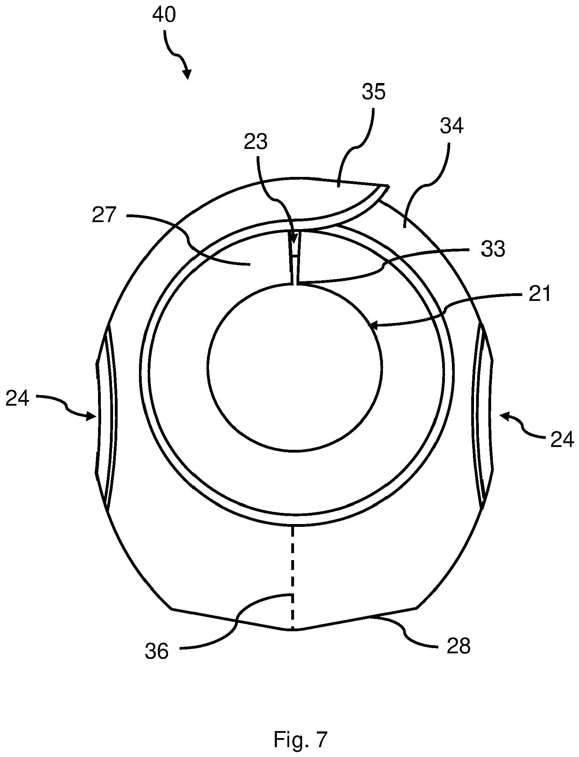

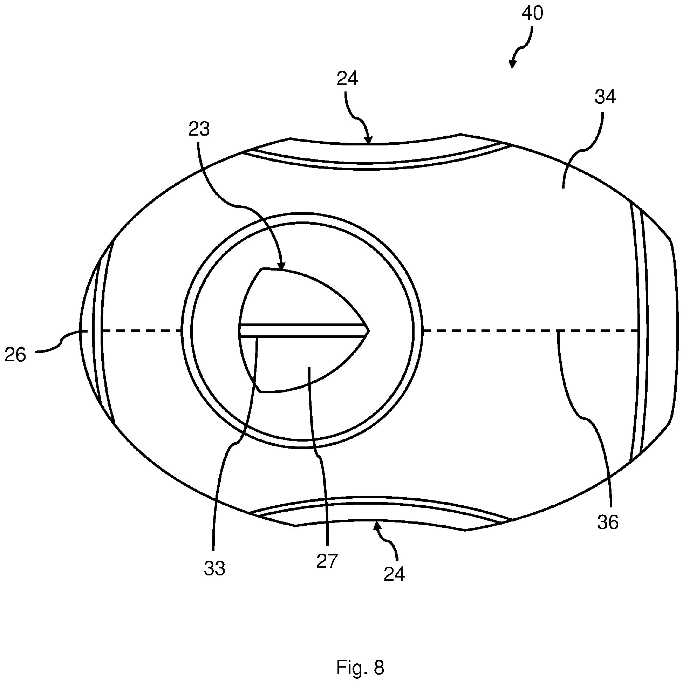

Dans toute la description, on appelle « supérieur » ou « haut » ce qui est en haut dans les figures 1, 2, 3 et 7 et « inférieur » ou « bas » ce qui est en bas dans ces figures. Les figures 1, 2, 3 et 7 représentent l'orientation du dispositif en cours d'utilisation par un patient allongé sur le dos. Les « hauteurs » découlent de ces définitions. Les « largeurs » sont définies de gauche à droite en figure 3, et de haut en bas en figure 4, 5, 6 et 8. Les « longueurs » sont définies de gauche à droite en figures 1, 2, 4, 5, 6 et 8.

On appelle « interne » ou « intérieur » ce qui est proche ou tourné vers le centre de la coque et « externe » ou « extérieur », ce qui est éloigné de ce centre ou tourné vers l'extérieur de la coque.

On note dès à présent que les figures 1 à 8 sont à l'échelle mais que leurs échelles peuvent être différentes.

On observe, sur les figures 1 à 5, un dispositif 20 de protection d'un pied de dimensions prédéterminées, notamment contre des escarres. Ce dispositif 20 comporte une coque en matériau élastiquement déformable à mémoire de forme. Cette coque possède un volume intérieur vide 31. Dans ce volume intérieur 31, débouchent des ouvertures atteignant la surface extérieure de la coque :
- une ouverture 21 dite « de jambe » propre à envelopper la base de la jambe au-dessus des chevilles et
- une ouverture 22 dite « de talon » dont au moins une section présente une forme globalement triangulaire dont un coin est orienté vers l'ouverture de jambe,
- une ouverture 23 dite « d'orteils » propre à laisser passer les cinq orteils du pied et
- deux ouvertures 24 dites « de malléoles » de part et d'autre d'un plan passant par les ouvertures 21, 22 et 23 de jambe, de talon et d'orteils (notamment le plan de coupe G-G de la figure 1, repéré en figure 3).

Les ouvertures 21 de jambe et 23 d'orteils sont reliées par des lèvres disjointes 33. La coque 20 en matériau élastiquement déformable à mémoire de forme. Préférentiellement, la coque est déformable sans déformation définitive jusqu'à un écartement des lèvres 33 d'au moins six centimètres sur toute leur longueur. L'écartement des lèvres 33 permet de faire entrer la cheville de l'utilisateur dans le volume intérieur 31. Pour obtenir cette déformation non définitive sous la seule force manuelle de l'utilisateur ou d'un praticien, l'homme du métier sélectionne un matériau ou une combinaison de matériaux, sa densité et son épaisseur dans l'ensemble de la coque, en fonction de son module d'élasticité (aussi appelé module d'Young) et de sa déformation limite de rupture.

La forme globalement triangulaire de l'ouverture de talon 22, visible en figures 4, 5 et 8 permet une meilleure répartition des forces autour du talon d'Achille. Bien entendu, cette forme globalement triangulaire de cette ouverture 22 peut présenter des côtés arrondis comme illustré dans les figures. Le sommet, ou coin, du triangle orienté vers l'ouverture de jambe correspond et supporte le talon d'Achille et son prolongement jusqu'au talon.

Cette forme triangulaire sert aussi à aider l'ouverture sans déformation définitive en réduisant l'épaisseur de la partie soumise à l'effort de compression lors de l'ouverture de la lèvre.

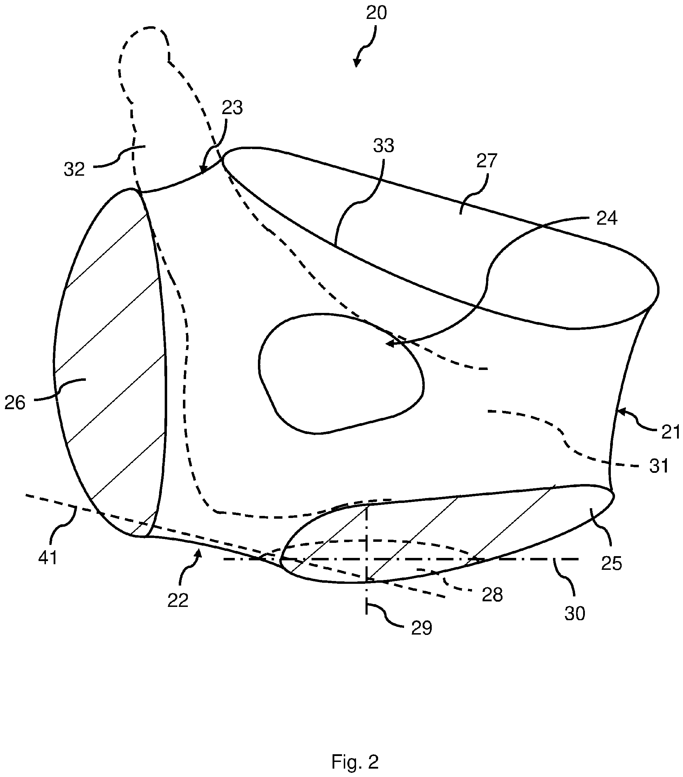

On note que la section triangulaire considérée est la section de surface minimale parallèle au plan 41 (figures 1 et 2) tangent au dispositif à l'embouchure extérieure de l'ouverture de talon 22.

On note aussi que les flancs de la coque au-dessus du sommet de ce triangle orienté vers l'ouverture de jambe, sont convexes et se rejoignent sur une arête adoucie 42 (figure 4). Ces flancs convexes ont, par exemple, une forme en tronc d'ovale dont la plus grande dimension est parallèle à l'axe allant du centre de l'ouverture de jambe 21 au centre de l'ouverture de talon 22. Par exemple, le rayon maximal de la section de cet ovale perpendiculaire à cet axe est compris entre trois et cinq centimètres et l'élongation de cet ovale parallèle à cet axe est comprise entre six et dix centimètres. L'arête concave qui relie ces flancs possède une section perpendiculaire à cet axe, par exemple circulaire, dont le plus petit rayon est, par exemple d'un centimètre. Ces flancs convexes soutiennent le bas de la jambe de part et d'autre du talon d'Achille.

Préférentiellement, l'arête 42 présente une extension (ou longueur) vers l'ouverture de jambe 21 pour que, lors d'un effort d'écartement des lèvres 33, l'arête 42 soit intégralement dans la zone en compression, c'est-à-dire du côté de l'ouverture de talon 22, par rapport à la ligne neutre 43 (figure 1) de séparation entre efforts de compression et efforts d'extension dans le matériau de la coque 20. L'arête 42 est donc soumise à un effort de compression et non d'extension, ce qui évite une déchirure.

On observe, en figure 2, une coupe de pied et cheville 32, en traits discontinus, montrant comment les dimensions de la coque sont adaptées aux dimensions prédéterminées du pied et de la cheville de l'utilisateur. Ainsi, pour chaque taille de pied 32, une coque et un dispositif 20 peuvent facilement être dimensionnés par l'homme du métier. Par exemple, la coque mesure entre 20 et 35 cm de longueur, entre 12 et 21 cm de largeur et entre 13 et 23 cm de hauteur pour les différentes tailles de pieds.

Le dispositif 20 permet de dégager et de protéger les zones à risques telles la face arrière du talon, les malléoles, la base du gros orteil, des pressions génératrices d'escarres et ce dans les diverses positions de l'utilisateur. De plus, le dispositif 20 protège le pied immobilisé 32 contre le risque d'équinisme.

Préférentiellement et comme illustré dans les figures 1 à 8, la coque 20 est symétrique par rapport à un plan G-G passant par les ouvertures 21, 22 et 23, de jambe, de talon et d'orteils. Ainsi, le dispositif peut être adapté, de manière indifférenciée, au pied droit comme au pied gauche. Dans des modes de réalisation (non représentés), la coque n'est pas symétrique et est configurée pour correspondre soit à un pied droit, soit à un pied gauche.

Pour assurer la souplesse de l'ouverture supérieure de la coque, préférentiellement, l'épaisseur des lèvres 33, mesurée dans un plan parallèle au plan G-G et passant par les ouvertures 21, 22 et 23, de jambe, de talon et d'orteils, est inférieure à trois centimètres.

Comme illustré en figures 1, 2, 5 et 7, la coque présente deux méplats 28 atteignant l'ouverture de talon 22. L'intersection des méplats 28 définit une première arête 37. L'intersection de chaque méplat 28 et de l'ouverture de talon 22 définit deux autres arêtes 38 plus courtes que la première arête 37. Les trois arêtes 37 et 38 formants un trièdre dont le sommet 39 est entre l'ouverture de talon 22 et l'ouverture de jambe 21. Le sommet 39 est sensiblement à équidistance du centre de l'ouverture de jambe 21 et du centre de l'ouverture d'orteils 23. L'angle entre les plans des méplats est de l'ordre de 20 à 40 degrés, ce qui permet au pied de prendre une position naturelle légèrement inclinée, de 10 à 20 degrés sur le côté par rapport à un plan vertical passant par l'axe de la jambe, ce qui augmente le confort de l'utilisateur.

Dans des modes de réalisation, tels que celui illustré dans les figures, les méplats 28 présentent, en dehors des arêtes 37 et 38, une périphérie sensiblement elliptique, dont l'axe 30 du plus grand diamètre est dans un plan passant par l'ouverture de jambe 21 et par l'ouverture de talon 22, par exemple un plan parallèle au plan G-G. L'axe 29 du plus petit diamètre de l'ellipse de chaque méplat 28 est dans un plan perpendiculaire au plan G-G.

Dans des modes de réalisation, les ouvertures 24 de malléoles sont cylindriques sur au moins une partie de la largeur de la coque.

Préférentiellement, la coque est constituée d'une mousse à cellules ouvertes de masse volumique comprise entre 30 et 40 kg/m³.

En revenant aux figures 1 et 2, on observe que la coupe G-G coupe la coque en une zone 25, entre l'ouverture de jambe 21 et l'ouverture de talon 22, et une zone 26 entre l'ouverture de talon 22 et l'ouverture d'orteils 23. En revanche, entre l'ouverture de jambe 21 et l'ouverture d'orteil 23, l'ouverture formée entre les parties 27, et entre les lèvres 33 est continue.

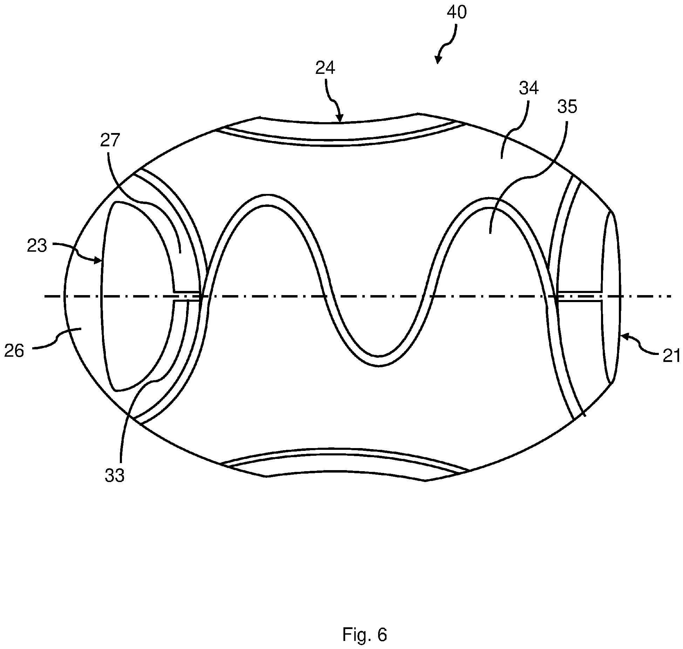

Dans le deuxième mode de réalisation 40 du dispositif, illustré en figures 6 à 8, ce dispositif 40 comporte, en plus de la coque, une housse 34 enveloppante amovible configurée pour entourer la coque en laissant accessible au moins l'ouverture de jambe 21 et l'ouverture d'orteils 23. Dans ce mode de réalisation, l'ouverture de talon 22 et les ouvertures de malléoles 24 sont aussi accessibles.

La housse 34 est formée de deux pièces reliées par une liaison 36, par exemple une couture ou une soudure. Des languettes 35 portant des morceaux de tissu autoagrippant (plus connu sous la marque déposée « Velcro ») permettent de refermer la housse 34 sur la coque. Ces languettes 35 permettent aussi d'adapter le serrage des lèvres 33 en fonction de la morphologie de l'utilisateur 32, une cheville et un pied 32 plus fins bénéficiant d'un serrage plus étroit.

On donne, ci-dessous, des exemples de matériaux pouvant être utilisés pour réaliser un dispositif 40.

La housse est en néoprène de deux centimètres d'épaisseur. Cette matière est assez douce pour ne pas créer de pression ou griffure, souple mais avec une certaine tenue pour une installation facilitée et une fermeture pérenne et correspondant à la bonne taille de pied. Le biais est une bande tissus élastique utile à la finition de la housse.

La housse comporte, par exemple, un tissu technique mousse et velours pour velcros contrecollé. La coque comporte, par exemple une mousse à cellules ouvertes, par exemple de polyuréthane (« PU ») de densité comprise entre 35 et 40 kg/m³. La coque est traitée par une peinture élastique configurée pour adhérer à la mousse de la coque et fermer les cellules de surface de la mousse de la coque.

Dans le cas d'une coque en mousse de polyuréthane, elle peut être traitée par une peinture élastique polyuréthane « sole vanter » mono composante à projeter lisse et satinée qui adhère à la mousse polyuréthane et forme une « peau » de surface. En variante, cette peinture ou la mousse formant la coque, est thermochromique. Elle comporte un adjuvant dont la couleur varie avec la température, notamment vers 37,5 °C ou 38 °C. Par exemple, cet adjuvant est de marque déposée « Naxagoras Technology ».

La peau de surface facilite l'utilisation d'une mousse simple par sa fermeture et son utilisation. Elle fiabilise la tenue du dispositif sur la jambe du patient, garantit les normes réglementaires concernant notamment la toxicité, l'innocuité pour le patient, le nettoyage et la désinfection.

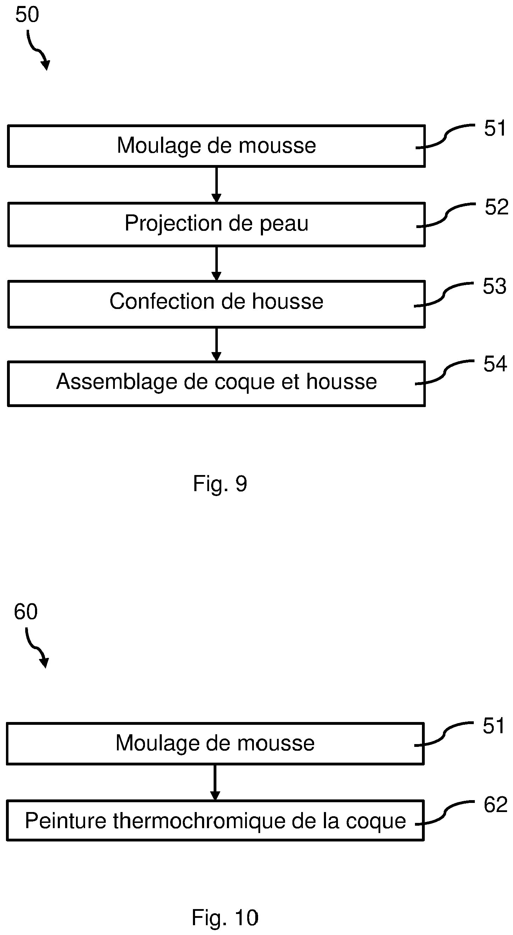

Comme illustré en figures 9 et 10, un procédé de fabrication d'un dispositif de protection d'un pied de dimensions prédéterminées, notamment contre des escarres, qui comporte une étape 51 de moulage d'une coque, entre deux demi-moules externes correspondant aux surfaces extérieures du dispositif, et autour d'un moule interne correspondant à un volume intérieur vide du dispositif.

Le moule interne comporte, jusqu'au contact avec le moule externe :
- une branche dite « de jambe » propre à envelopper la base de la jambe au-dessus des chevilles et
- une branche dite « d'orteils » propre à laisser passer les cinq orteils du pied, les branches de jambe et d'orteils étant reliées par une nervure de séparation de lèvres, nervure qui rejoint les moules externes,
- une branche dite « de talon » dont au moins une section présente une forme globalement triangulaire dont un coin est orienté vers l'ouverture de jambe et
- de deux branches dites « de malléoles » de part et d'autre d'un plan passant par les branches de jambe, d'orteils et de talon.

Le moulage de la coque se fait dans un matériau élastiquement déformable à mémoire de forme configuré pour que la coque soit déformable sans déformation définitive jusqu'à un écartement des lèvres d'au moins six centimètres sur toute leur longueur.

Le moulage est effectué, par exemple, par injection à froid de bi-composant avec douze heures de repos dans un moule en trois parties :
- le moule intérieur en forme du pied avec une rainure pour constituer l'espace entre les lèvres 33 et des excroissances préférentiellement cylindriques sur une partie de leur élongation, pour former les ouvertures de malléoles,
- deux flancs concaves correspondant aux surfaces extérieures de la coque,
- un support du moule intérieur qui l'immobilise entre les flancs.

A la fin du durcissement de la mousse, on retire les deux moules externes puis le moule interne entre les lèvres 33, comme un pied lors de l'utilisation par un patient.

Dans le mode de réalisation 50 du procédé illustré en figure 9, au cours d'une étape optionnelle 52, on effectue une projection de matériau constituant la peau, par exemple du polyuréthane liquide. Ce matériau constitue une peinture élastique configurée pour adhérer à la mousse de la coque et fermer les cellules de surface de la mousse de la coque. Ce matériau a aussi l'avantage d'être lavable par lissage, souple, désinfectable, renforcé sur face, sec en douze heures et de présenter un aspect lisse.

Au cours d'une étape 53, on confectionne la housse, avec découpe et couture de néoprène, biais et scratches.

Au cours d'une étape 54, on assemble la housse sur la coque.

Dans le mode de réalisation 60 du procédé illustré en figure 10, au cours d'une étape 62, on enduit, par exemple par projection, la coque avec une peinture thermochromique. Bien entendu, la peinture thermochromique peut être configurée pour adhérer à la mousse de la coque et fermer les cellules de surface de la mousse de la coque, être lavable, souple, désinfectable, et présenter un aspect lisse.

En variante de chacun des modes de réalisation illustrés en figures 10 et 11, au cours de l'étape de moulage 51, la mousse est thermochromique et comporte un adjuvant dont la couleur varie avec la température.

## Revendications

1. Procédé (50, 60) de fabrication d'un dispositif de protection d'un pied de dimensions prédéterminées, notamment contre des escarres, qui comporte une étape (51) de moulage d'une coque (20), entre deux demi-moules externes correspondant aux surfaces extérieures du dispositif, et autour d'un moule interne correspondant à un volume intérieur vide du dispositif et comportant jusqu'au contact avec le moule externe :
- une branche dite « de jambe » propre à envelopper la base de la jambe au-dessus des chevilles,
- une branche dite « d'orteils » propre à laisser passer les cinq orteils du pied, les branches de jambe et d'orteils étant reliées par une nervure de séparation de lèvres, nervure qui rejoint les moules externes,
- une branche dite « de talon » dont au moins une section présente une forme globalement triangulaire dont un coin est orienté vers l'ouverture de jambe et
- deux branches dites « de malléoles » de part et d'autre d'un plan passant par les branches de jambe, d'orteils et de talon ;
le moulage de la coque se faisant dans un matériau élastiquement déformable à mémoire de forme.

2. Procédé (50, 60) dans lequel, lors de l'étape de moulage (51), le matériau élastiquement déformable est configuré pour que la coque soit déformable sans déformation définitive jusqu'à un écartement des lèvres suffisant pour le passage du moule interne entre ces lèvres.

3. Procédé (50, 60) selon l'une des revendications 1 ou 2, qui comporte, de plus, une étape de confection (53) d'une housse (34) et une étape d'assemblage (54) de la coque (20) et la housse, la housse étant enveloppante, amovible et configurée pour entourer la coque en laissant accessible au moins l'ouverture de jambe (21) et l'ouverture d'orteils (23).

4. Procédé (50, 60) selon l'une des revendications 1 à 3, dans lequel lors de l'étape de moulage (51), la coque (20) formée présente deux méplats (28) atteignant l'ouverture de talon (22), l'intersection des méplats définissant une première arête (37) et l'intersection de chaque méplat et de l'ouverture de talon définissant deux autres arêtes (38) plus courtes que la première arête, les trois arêtes formant un trièdre dont le sommet (39) est entre l'ouverture de talon (22) et l'ouverture de jambe (21) et sensiblement à équidistance du centre de l'ouverture de jambe et du centre de l'ouverture d'orteils (23), les méplats (28) formés présentant, en dehors des arêtes (37, 38), une périphérie sensiblement elliptique, dont l'axe (30) du plus grand diamètre est dans un plan passant par l'ouverture de jambe (21) et par l'ouverture de talon (22).

5. Procédé (50, 60) selon l'une des revendications 1 à 4, dans lequel le matériau élastiquement déformable à mémoire de forme est une mousse à cellules ouvertes de masse volumique comprise entre 30 et 40 kg/m³.

6. Procédé (50, 60) selon la revendication 9, qui comporte, de plus, une étape de projection (52) d'une peinture élastique sur la coque (20) formée, la peinture élastique étant configurée pour adhérer à la mousse de la coque et fermer les cellules de surface de la mousse de la coque.

7. Procédé (50, 60) selon l'une des revendications 1 à 6, dans lequel, au cours de l'étape de moulage (51), la mousse est thermochromique qui comporte un adjuvant dont la couleur varie avec la température.

8. Dispositif (20, 40) moulé de protection d'un pied (32) obtenu par le procédé (50, 60) selon l'une des revendications 1 à 7, qui comporte une coque (20) en matériau élastiquement déformable à mémoire de forme, coque dotée d'un volume intérieur vide (31) et, débouchant dans ce volume :
- une ouverture (21) dite « de jambe » propre à envelopper la base de la jambe au-dessus des chevilles,
- une ouverture (23) dite « d'orteils » propre à laisser passer les cinq orteils du pied,
les ouvertures de jambe et d'orteils étant reliées par des lèvres (33) disjointes, la coque étant déformable élastiquement sans déformation définitive jusqu'à un écartement des lèvres d'au moins six centimètres sur toute leur longueur,
- une ouverture (22) dite « de talon » dont au moins une section présente une forme globalement triangulaire dont un coin est orienté vers l'ouverture de jambe et
- deux ouvertures (24) dites « de malléoles » de part et d'autre d'un plan (G-G) passant par les ouvertures de jambe, d'orteils et de talon.

9. Dispositif (20, 40) selon la revendication 8, dans lequel la coque (20) est symétrique par rapport à un plan (G-G) passant par les ouvertures de jambe (21), d'orteils (23) et de talon (22).

10. Dispositif (40) selon l'une des revendications 8 ou 9, qui comporte, de plus, une housse (34) enveloppante amovible configurée pour entourer la coque (20) en laissant accessible au moins l'ouverture de jambe (21) et l'ouverture d'orteils (23).

11. Dispositif (20, 40) selon l'une des revendications 8 à 10, dans lequel la coque (20) présente deux méplats (28) atteignant l'ouverture de talon (22), l'intersection des méplats définissant une première arête (37) et l'intersection de chaque méplat et de l'ouverture de talon définissant deux autres arêtes (38) plus courtes que la première arête, les trois arêtes formant un trièdre dont le sommet (39) est entre l'ouverture de talon (22) et l'ouverture de jambe (21) et sensiblement à équidistance du centre de l'ouverture de jambe et du centre de l'ouverture d'orteils (23), les méplats (28) présentant, en dehors des arêtes (37, 38), une périphérie sensiblement elliptique, dont l'axe (30) du plus grand diamètre est dans un plan passant par l'ouverture de jambe (21) et par l'ouverture de talon (22).

12. Dispositif (20, 40) selon l'une des revendications 8 à 11, dans lequel la coque (20) est constituée d'une mousse à cellules ouvertes de masse volumique comprise entre 30 et 40 kg/m³.

13. Dispositif (20, 40) selon la revendication 12, dans lequel la coque (20) est traitée par une peinture élastique configurée pour adhérer à la mousse de la coque et fermer les cellules de surface de la mousse de la coque.

14. Dispositif (20, 40) selon la revendication 13, dans lequel la peinture est thermochromique et comporte un adjuvant dont la couleur varie avec la température.

15. Dispositif (20, 40) selon l'une des revendications 8 à 14, dans lequel la mousse formant la coque est thermochromique et comporte un adjuvant dont la couleur varie avec la température.
